# EUROPÄISCHE PATENTSCHRIFT

(11) **EP 2 331 037 B1**
(45) Veröffentlichungstag und Bekanntmachung des Hinweises auf die Patenterteilung: **08.02.2012**
(21) Anmeldenummer: 09767978.1
(22) Anmeldetag: 04.12.2009
(51) Int. Cl.: A61F 7/08, A61B 19/00

(54) **BEFÜLLSTÄNDER FÜR WÄRMFLASCHEN**
FILLING STAND FOR HOT WATER BOTTLES
SUPPORT DE REMPLISSAGE DE BOUILLOTTES

(30) Priorität: 09.12.2008 DE 102008063544
(43) Veröffentlichungstag der Anmeldung: 15.06.2011
(73) Patentinhaber: Fashy GmbH Produktion und Vertrieb, 70825 Korntal-Münchingen (DE)
(72) Erfinder: KRAUS, Alexander, 71229 Leonberg (DE)
(74) Vertreter: Dreiss
(86) Internationale Anmeldenummer: PCT/EP2009/008678
(87) Internationale Veröffentlichungsnummer: WO 2010/066383

(56) Entgegenhaltungen:
- DE-A1- 3 511 096
- DE-U1- 20 108 609
- DE-U1-202008 006 334

## Beschreibung

Die vorliegende Erfindung bezieht sich auf einen Befüllständer für Wärmflaschen nach dem Oberbegriff des Anspruchs 1.

Bei einer derartigen aus der DE 20 2008 006334 U1 bekannten Halterung zum Befüllen von Wärmflaschen sind der Boden und die Begrenzungswände, die parallel zueinander aufrechtstehend auf dem Boden angeschweißt verlaufen, jeweils aus Metallplatten. Der Abstand der beiden Begrenzungsplatten ist etwas größer als der Dicke des unbefüllten Wärmflaschenkorpus entspricht. Am oberen Bereich der Begrenzungswände ist die Halteaufnahme in Form eines Drahtbügels befestigt, in den die Wärmflasche mit ihrem Füllstutzen einhängbar ist. Abgesehen davon, dass dieser metallische Befüllständer relativ schwer und herstellungstechnisch aufwändig ist, beschränken die beiden parallelen Begrenzungsplatten das Füllvolumen erheblich. Beim Herausnehmen der befüllten Wärmflasche muss diese bewusst festgehalten werden, da das Wasser nach unten schwappt und die Wärmflasche ausbaucht. Diese Kraft macht auch das Herausnehmen der Wärmflasche aus dem Ständer schwierig.

Aufgabe der vorliegenden Erfindung ist es deshalb, einen Befüllständer für Wärmflaschen der eingangs genannten Art zu schaffen, der mit weniger Bauteilen auskommt, der eine größere Befüllmenge zulässt und bei dem die Entnahme der befüllten Wärmflasche einfacher ist.

Zur Lösung dieser Aufgabe sind bei einem Befüllständer für Wärmflaschen der genannten Art die im Anspruch 1 angegebenen Merkmale vorgesehen.

Durch die erfindungsgemäßen Maßnahmen ist eine vereinfachte Herstellung erreicht, da das gesonderte Bauteil einer Halteaufnahme für den Füllstutzen einer Wärmflasche entfällt. Des weiteren ist aufgrund des in seinem lichten Abstand erweiterten unteren Bereichs ein sicherer Stand mit befüllter Wärmflasche und die Möglichkeit gegeben, die Wärmflasche zwar ebenfalls mit einem begrenzten jedoch höheren Füllvolumen zu befüllen, das darüber hinaus variabel sein kann und das bis Gewinde-Unterkante reicht. Außerdem ist dadurch aufgrund verringerter Reibung zwischen Wärmflasche und Befüllständer die Entnahme einfacher. Wird die Wärmflasche erst verschlossen, bevor sie aus dem Befüllständer entnommen wird, kann keine Luft entweichen. Ferner kann es zweckmäßig sein, die Merkmale gemäß Anspruch 2 vorzusehen, was zu einem Füllvolumen von etwa zwei Drittel bis drei Viertel führt, wobei gleichzeitig gewährleistet ist, dass die Luft aus dem Korpus der Wärmflasche im Wesentlichen vollständig entweichen kann, wenn sie im Befüllständer bis zur Unterkante des Verschlussgewindes befüllt wird.

Vorteilhafte Ausgestaltungen der Halteaufnahme und des oberen Endbereichs der Begrenzungswände ergeben sich aus den Merkmalen eines oder mehrerer der Ansprüche 3 bis 4. Die Ausgestaltung der Halteaufnahme ist dabei von besonderer Einfachheit.

Mit den Merkmalen gemäß Anspruch 5 ist erreicht, dass die Halteaufnahme sich beim Einhängen der Wärmflasche mittels Füllstutzen und während des Befüllens der Wärmflasche nicht aufweiten kann und damit der Einfüllstutzen sicher gehalten ist Zweckmäßigerweise sind dabei die Merkmale nach Anspruch 6 vorgesehen.

In weiterer Ausgestaltung sind die Merkmale gemäß Anspruch 7 vorgesehen, was zu einer Erhöhung der Stabilität des Befüllständers führt.

Gemäß einem weiteren Ausführungsbeispiel sind die Merkmale gemäß Anspruch 8 vorgesehen, was den Vorteil hat, dass der Befüllständer auch als Aufbewahrständer für die Wärmflasche Verwendung finden kann, wobei in vorteilhafter Weise der Verschluss gesondert vom Wärmflaschenkorpus am Befüllständer selbst aufbewahrt werden kann. Damit ist auch gewährleistet, dass die Wärmflasche zum Austrocknen ständig belüftet ist. Außerdem kann die so im Befüllständer aufbewahrte Wärmflasche zu Dekorationszwecken, beispielsweise mittels Aufnahme von künstlichen oder echten Blumen verwendet werden. Beilspielsweise können hierfür die in Anspruch 9 vorgesehenen Aufbewahrungsorte für den Verschluss vorgesehen sein.

Zum bequemen Ergreifen des Befüllständers mit oder ohne befüllter oder nicht befüllter Wärmflasche sind die Merkmale nach Anspruch 10 vorgesehen. Zum einfachen Handhaben für Rechts- und Linkshänder gleichermaßen sind die Merkmale nach Anspruch 11 vorgesehen.

Die Herausnahme von befüllten Wärmflaschen, die einen textilen Überzug besitzen, aus dem Befüllständer ist ohne Überwindung merklicher Reibung ohne weiteres möglich. Werden jedoch Wärmflaschen aus Gummi, Silikon, Thermoplast oder dergleichen im befülltem Zustand aus dem Befüllständer herausgezogen, muss insbesondere diejenige Reibung überwunden werden, die sich zwischen dem Wärmflaschenkorpus und zudem oberen Bereich der Begrenzungswände aufbaut. Um dies zu vermeiden, sind die Merkmale gemäß Anspruch 12 vorgesehen, die zumindest zu einer erheblichen Reduzierung des Reibungskoeffizienten und damit der Reibung beim Herausnehmen der befüllten Wärmflasche aus dem Befüllständer führt.

Der Befüllständer ist zweckmäßigerweise gemäß den Merkmalen nach Anspruch 13 einem schlag- und bruchfesten Kunststoff hergestellt. Dabei kann gemäß den Merkmalen des Anspruchs 14 ein glasklarer oder farblich getönter Kunststoff oder auch ein bedruckter oder strukturierter Kunststoff Verwendung finden. Der Befüllständer kann damit-nicht heiß werden.

Vorteilhafte Herstellungstechniken ergeben sich dabei aus den Merkmalen nach Anspruch 5.

Weitere Einzelheiten der Erfindung sind der folgenden Beschreibung zu entnehmen, in der die Erfindung anhand des in der Zeichnung dargestellten Ausführungsbeispieles näher beschrieben und erläutert ist. Es zeigen:

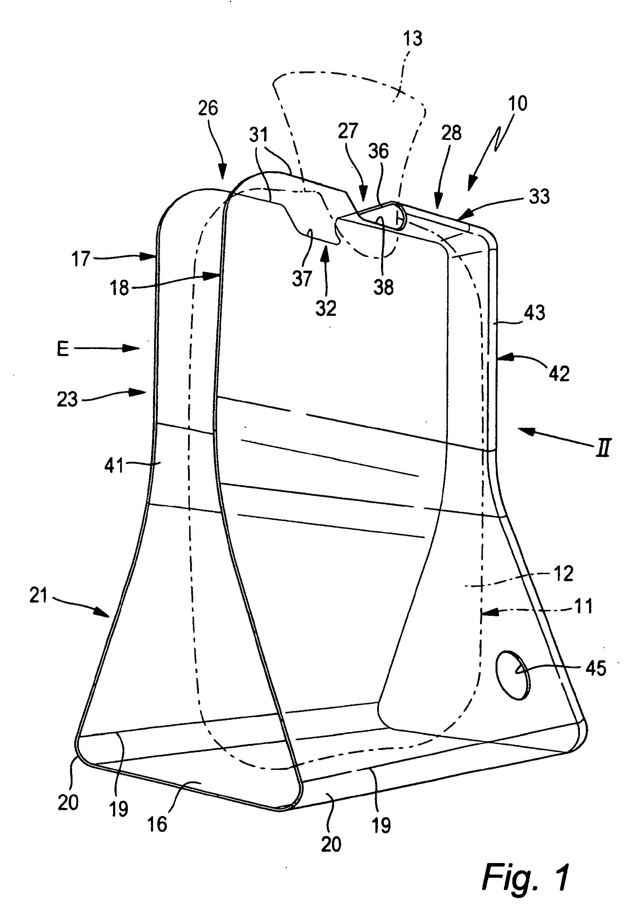

- Figur 1: in perspektivischer schematischer Darstellung einen Befüllständer für Wärmflaschen gemäß einem bevorzugten Ausführungsbeispiel vorliegender Erfindung und
- Figur 2: eine Seitenansicht des Befüllständers gemäß Pfeil II der Figur 1, gemäß einer Variante.

Der in der Zeichnung dargestellte Befüllständer 10 dient zur Aufnahme einer strichpunktiert dargestellten üblichen Wärmflasche 11, die aus einem Korpus 12, einem mit dem Korpus dicht verbundenen Füllstutzen 13 und einem nicht dargestellten, in den Füllstutzen 13 ein- und ausschraubbarem Verschluss besteht. Der Befüllständer 10 dient sowohl zum Halten der Wärmflasche 11 während deren Befüllen als auch zum Aufbewahren einer leeren Wärmflasche 11.

Der Befüllständer 10 ist aus einem schlag- und/oder bruchfesten Kunststoffmaterial einstückig hergestellt, und zwar entweder durch Spritzgießen oder durch Tiefziehen.

Der Befüllständer besteht im Wesentlichen aus einem Boden 16, der für die Standfestigkeit sorgt und zwei einander gegenüberliegenden Begrenzungswänden 17 und 18, die an ihrem unteren Ende 19 über bogenförmige Konturen 20 in den Boden 16 einstückig übergehen.

Die beiden Begrenzungswände 17 und 18 verlaufen in einem unteren Bereich 21 ausgehend von etwa der Breite des rechteckförmigen Bodens 16 konisch aufeinander zu, so dass sich ihr lichter Abstand zu einem oberen Bereich 23 hin verringert. Der untere Bereich 21 der Begrenzungswände 17 und 18 geht konkav mittels eines großen Radius in den oberen Bereich 23 über, in welchem die beiden Begrenzungswände 17 und 18 einen im Wesentlichen konstanten lichten Abstand besitzen. Dieser konstante lichte Abstand ist erheblich kleiner als die Breite des Bodens 16 und ist etwas größer als der Dicke eines unbefüllten Korpus 12 einer Wärmflasche 11 entspricht. Die Ausdehnung bzw. Längen von unterem Bereich 21 und oberem Bereich 23 in Richtung der Längserstreckung des Befüllständers 10 sind etwa gleich groß, so dass die Wärmflasche 11 bzw. deren Korpus 12 bis zum Verschlussrand im Füllstutzen 13 hin etwa zu zwei Drittel bis drei Viertel gefüllt werden kann, wobei gleichzeitig die Luft im Korpus 12 im Wesentlichen vollständig entweicht. Es versteht sich, dass durch Änderung der Höhenverhältnisse von unterem Bereich 21 und oberem Bereich 23 die Befüllmenge einer im Befüllständer 10 gehaltene Wärmflasche 11 veränderbar ist.

Das obere Ende der Begrenzungswände 17 und 18 ist über deren Breite unterschiedlich ausgebildet. Der in Einschubrichtung E der unbefüllten Wärmflasche 11 in den Befüllständer 10 vordere Bereich 26 ist als eben auslaufende Kante 31 ausgebildet. In nicht dargestellter Weise kann die Kante 31 auch jeweils nach innen (aufeinander zu) abgekantet sein. Der sich daran anschließende mittlere Bereich 27 ist als Halteaufnahme 32 ausgebildet. An die Halteaufnahme 32 schließt sich ein in Einschubrichtung E hinterer Bereich 28 an, in welchem die beiden Begrenzungswände 17 und 18 einstückig miteinander verbunden sind. Dieser Verbindungsbereich 33 ist gewölbt, so dass er als eine zur Halteaufnahme 32 hinweisende Anschlag- bzw. Anlagekante 36 für den Füllstutzen 13 der Wärmflasche 11 dient. Die Halteaufnahme 32 ist durch jeweils eine Ausnehmung bzw. Auskerbung 37 bzw. 38 in der oberen Kante 31 der Begrenzungswände 17, 18 gebildet. Die von der betreffenden Kante 31 senkrecht und in Längsrichtung der Begrenzungswand 17 bzw. 18 ausgehende Ausnehmung 37 bzw. 38 besitzt eine dem Füllstutzen 13 entsprechende Breite und eine Tiefe, die entweder eine frei hängende oder eine auf dem Boden 16 aufsitzende Anordnung der befüllten Wärmflasche 11 im Befüllständer 10 zulässt, welche letztere zur einer erhöhten Standsicherheit führt.

Die Breite des Befüllständers 10 ist gleich der oder etwas größer als die Breite einer handelsüblichen Wärmflasche 11, wobei die Halteaufnahme 32 etwa in der Quermitte des Befüllständers 10 vorgesehen ist. Dadurch wird die Wärmflasche 11 insgesamt im Befüllständer 10 aufgenommen. Es ist auch möglich den Befüllständer 10 etwas schmaler auszuführen, so dass die Wärmflasche 11 mit einer Längskante etwas über die Begrenzungswände 17, 18 bzw. die Stirn 41 ragt.

In Einschubrichtung E, d.h. an seiner vorderen Stirn 41, ist der Befüllständer 10 naturgemäß offen. Diese Einschuböffnung kann im oberen Bereich 23 etwas konisch erweitert sein. Beim dargestellten Ausführungsbeispiel ist die in Einschubrichtung hintere Stirn 42 mit einer Stirnwand 43 verschlossen. Die Stirnwand 43 ist mit dem Boden 16 und mit den beiden Begrenzungswänden 17 und 18 einstückig verbunden, so dass sie eine entsprechend verjüngende flaschenähnliche Form aufweist. Diese Stirnwand 43 dient der Stabilisierung bzw. Standfestigkeit des Befüllständers 10. Sie geht am oberen Ende in den Verbindungsbereich 33 entsprechend der Form des Korpus 12 gerundet über. Der Boden 16 kann von hinten nach vorne leicht konisch erweiternd sein, um das Ausformen aus der Herstellungsform zu erleichtern.

Beim in der Zeichnung dargestellten Ausführungsbeispiel ist im unteren Bereich 21 der Begrenzungswand 17 eine kreisrunde Öffnung 45 vorgesehen, die so groß ist, dass sie das Gewindeende des nicht dargestellten Verschlusses entweder einschraubend oder leicht rastend einsteckbar aufnimmt. Es versteht sich, dass die kreisrunde Öffnung 45 statt dessen in der anderen Begrenzungswand 18 oder auch in beiden Begrenzungswänden 17, 18 vorgesehen sein kann. In diesem Bereich ist sowohl bei nicht befüllter als auch bei befüllter Wärmflasche 11 ausreichend Platz für das Aufbewahren eines Verschlusses vorhanden. Es versteht sich, dass es auch möglich ist, die kreisrunde Öffnung 45 im unteren Bereich der Stirnwand 43 vorzusehen.

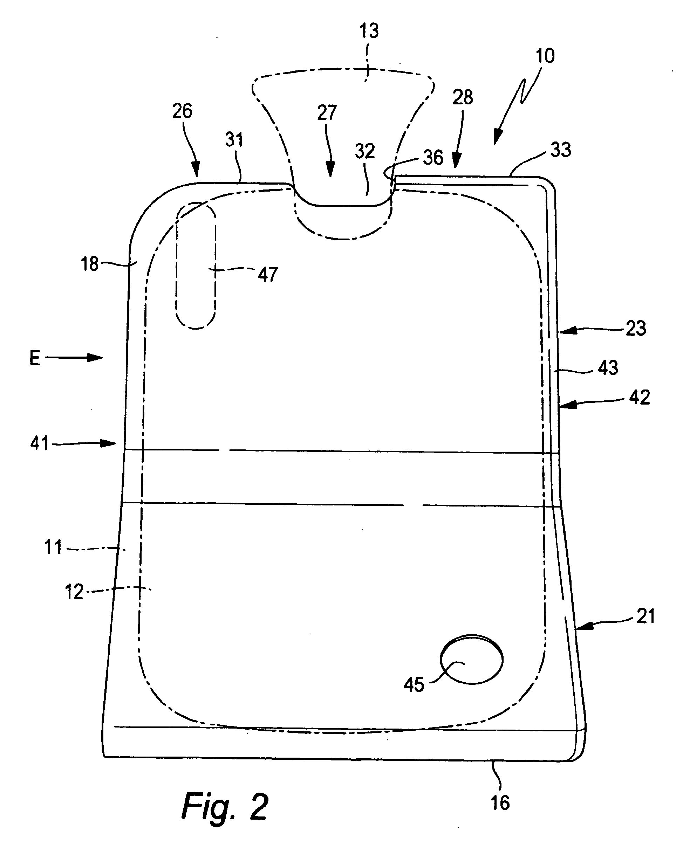

Gemäß Figur 2 ist gestrichelt eine Variante insofern vorgesehen als diese eine Griffausnehmung 47 in einer oder beiden Begrenzungswänden 17 und/oder 18 gleichermaßen umfasst. In bevorzugter Weise sind die Griffausnehmungen 47 in beiden Begrenzungswänden 17 und 18 identisch ausgebildet und zwar länglich rechteckförmig und abgerundet zur Aufnahme nicht nur des Daumens sondern auch von drei oder vier Fingern gegenüber dem Daumen der Hand einer Bedienungsperson. Die Griffausnehmung 47 sind an einander gegenüberliegenden Stellen in einem oberen Drittel der Begrenzungswände 17, 18 nahe den oberen Kanten 31 sowie den offenen Stirnkanten 41 vorgesehen. Die Griffausnehmungen 47 sind parallel zur Stirnkante 41 verlaufend ausgebildet.

Wärmflaschen 11, die mit einem textilen Überzug versehen sind, sind in befülltem Zustand in einfacher Weise aus dem Befüllständer 10 auch dann herauszuziehen, wenn ihr Korpus 12 im oberen Bereich 23 der dort parallelen Begrenzungswände 17 und 18 reibend anliegt. Um dies auch bei Wärmflaschen 11 zu gewährleisten, die keinen textilen Überzug besitzen und die aufgrund ihres verwendeten Kunststoffes oder Gummis an den Innenflächen der Begrenzungswände 17 und 18 reiben, sind in nicht dargestellter Weise die Innenflächen der Begrenzungswände 17 und 18 zumindest im oberen Bereich 23 mit reibungsmindernden Maßnahmen versehen, wie beispielsweise mit einer entsprechenden z.B. Gleitmittel-Beschichtung oder mit gerauten sandgestrahlten o. dgl. Anlageflächen versehen. Diese Maßnahmen können an allen Innenflächen einschließlich des Bodens vorgesehen sein.

Der Befüllständer 10 ist beispielsweise aus einem Kunststoff, wie beispielsweise PETG, Polycarbonat, PP, Polystyrol, ABS, Acryl oder dergleichen.

Der Kunststoff kann beispielsweise glasklar, matt oder opaque oder farblich getönt sein. Es ist möglich, den Kunststoff mit Strukturen zu versehen und/oder einzufärben. Außerdem kann ein Kunststoff verwendet werden, der bedruckbar ist oder der mit Prägungen versehen oder bildkaschiert oder strukturiert werden kann.

## Patentansprüche

1. Befüllständer (10) für Wärmflaschen (11), mit einem Boden (16) und zwei seitlichen, im Abstand angeordneten und mit dem Boden (16) verbundenen Begrenzungswänden (17, 18) deren lichter Abstand vom Boden (16) zur Halteaufnahme (32) hin sich in einem unteren Bereich (21) verringert und in einem oberen Bereich (23) konstant bleibt und mit einer Halteaufnahme (32) für den Füllstutzen (13) der Wärmflasche (11), **dadurch gekennzeichnet, dass** Boden (16) und Begrenzungswände (17, 18) einstückig geformt sind, und dass die Halteaufnahme (32) für den Füllstutzen (13) durch obere freie Kantenbereiche (31) der Begrenzungswände (17, 18) gebildet ist, welche Kantenbereiche (31) mit einer etwa rinnenförmigen Ausnehmung (37, 38) versehen sind.

2. Befüllständer nach Anspruch 1, **dadurch gekennzeichnet, dass** der untere Bereich (21) und der obere Bereich (23) der Begrenzungswände (17, 18) etwa dieselbe Länge aufweisen.

3. Befüllständer nach Anspruch 1 oder 2, **dadurch gekennzeichnet, dass** die Kantenbereiche (31) der Begrenzungswände (17, 18) in Einschubrichtung (E) der Wärmflasche (11) zwischen die Begrenzungswände (17, 18) geradlinig nach oben auslaufen.

4. Befüllständer nach Anspruch 1 oder 2, **dadurch gekennzeichnet, dass** die Kantenbereiche (31) der Begrenzungswände (17, 18) in Einschubrichtung (E) der Wärmflasche (11) zwischen die Begrenzungswände (17, 18) nach innen aufeinander zu abgekantet sind.

5. Befüllständer nach mindestens einem der vorhergehenden Ansprüche, **dadurch gekennzeichnet, dass** die Begrenzungswände (17, 18) in Einschubrichtung (E) der Wärmflasche (11) in einem der Halteaufnahme (32) hinteren Bereich (28) miteinander verbunden sind und ggf. der im hinteren Bereich (28) vorgesehene obere Verbindungsbereich (33) der beiden Begrenzungswände (17, 18) an die Halteaufnahme (32) angrenzt.

6. Befüllständer nach mindestens einem der vorhergehenden Ansprüche, **dadurch gekennzeichnet, dass** die Halteaufnahme (32) quermittig an den Begrenzungswänden (17, 18) angeordnet ist.

7. Befüllständer nach mindestens einem der vorhergehenden Ansprüche, **dadurch gekennzeichnet, dass** die Begrenzungswände (17, 18) und der Boden (16) an einer der offenen stirnseitigen Einschubseite (41) abgewandten Stirnseite (42) mittels einer Stirnwand (43) verbunden sind, wobei Boden (16) und Begrenzungswände (17, 18) sowie Stirnwand (43) vorzugsweise einstückig geformt sind.

8. Befüllständer nach mindestens einem der vorhergehenden Ansprüche, **dadurch gekennzeichnet, dass** eine Einstecköffnung (45) für einen Verschluss der Wärmflasche (11) vorgesehen ist.

9. Befüllständer nach Anspruch 8, **dadurch gekennzeichnet, dass** die Einstecköffnung (45) im unteren Bereich (21) einer oder beider Begrenzungswände (17, 18), vorzugsweise am Einschubende, oder im oberen Verbindungsbereich (33) der Begrenzungswände (17, 18) oder im unteren Bereich der Stirnwand (43) vorgesehen ist.

10. Befüllständer nach mindestens einem der vorhergehenden Ansprüche, **dadurch gekennzeichnet, dass** vorzugsweise beide Begrenzungswände (17, 18) an einander gegenüberliegenden Stellen in einem oberen den oberen Kanten (31) sowie den offenen Stirnkanten (41) zugewandten Bereich mit Griffausnehmungen (47) versehen sind.

11. Befüllständer nach Anspruch 10, **dadurch gekennzeichnet, dass** die Griffausnehmungen (47) länglich rechteckförmig und parallel zur offnen Stirnkante (41) verlaufend und an beiden Begrenzungswänden (17, 18) vorzugsweise identisch ausgebildet sind.

12. Befüllständer nach mindestens einem der vorhergehenden Ansprüche, **dadurch gekennzeichnet, dass** die Begrenzungswände (17, 18) zumindest in ihrem etwa parallelen oberen Bereich (23) innenseitig mit reibungsmindernden Maßnahmen, wie Gleitmittel-Beschichtung oder dergleichen versehen oder aufgerauht, sandgestrahlt o. dgl. ist.

13. Befüllständer nach mindestens einem der vorhergehenden Ansprüche, **dadurch gekennzeichnet, dass** er aus schlag- und/oder bruchfestem Kunststoff, beispielweise PETG, Polycarbonat, PP, Polystyrol, ABS, Acryl, geformt ist.

14. Befüllständer nach Anspruch 13, **dadurch gekennzeichnet, dass** ein glasklarer matter, opaquer oder farblich getönter Kunststoff oder ein strukturierter Kunststoff verwendet ist.

15. Befüllständer nach mindestens einem der vorhergehenden Ansprüche, **dadurch gekennzeichnet, dass** er gespritzt oder durch ein Tiefziehteil gebildet ist.

## Claims

1. A filling rack (10) for hot-water bottles (11) including a bottom (16) and two lateral, spaced boundary walls (17, 18) connected to the bottom (16), the clearance of which decreases in a lower region (21) from the bottom (16) towards the retaining receptacle (32) and remains constant in an upper region (23), and including a retaining receptacle (32) for the filling nozzle (13) of the hot-water bottle (11), **characterized in that** the bottom (16) and boundary walls (17, 18) are integrally formed and that the retaining receptacle (32) for the filling nozzle (13) is formed by upper free edge regions (31) of the boundary walls (17, 18), which edge regions (31) are provided with an approximately groove-shaped recess (37, 38).

2. The filling rack according to claim 1, **characterized in that** the lower region (21) and the upper region (23) of the boundary walls (17, 18) have approximately the same length.

3. The filling rack according to claim 1 or 2, **characterized in that** the edge regions (31) of the boundary walls (17, 18) extend linearly towards the top in the insertion direction (E) of the hot-water bottle (11) between the boundary walls (17, 18).

4. The filling rack according to claim 1 or 2, **characterized in that** the edge regions (31) of the boundary walls (17, 18) are inwardly chamfered towards each other in the insertion direction (E) of the hot-water bottle (11) between the boundary walls (17, 18).

5. The filling rack according to at least one of the preceding claims, **characterized in that** the boundary walls (17, 18) are connected to each other in the insertion direction (E) of the hot-water bottle (11) in a region (28) rearward of the retaining receptacle (32), and optionally the upper connection region (33) of the two boundary walls (17, 18) provided in the rear region (28) adjoins to the retaining receptacle (32).

6. The filling rack according to at least one of the preceding claims, **characterized in that** the retaining receptacle (32) is transversely centrally disposed on the boundary walls (17, 18).

7. The filling rack according to at least one of the preceding claims, **characterized in that** the boundary walls (17, 18) and the bottom (16) are connected by means of an end wall (43) at an end side (42) facing away from the open front-end insertion side (41), wherein bottom (16) and boundary walls (17, 18) as well as end wall (43) are preferably integrally formed.

8. The filling rack according to at least one of the preceding claims, **characterized in that** an insertion opening (45) for a closure of the hot-water bottle (11) is provided.

9. The filling rack according to claim 8, **characterized in that** the insertion opening (45) is provided in the lower region (21) of one or both boundary walls (17, 18), preferably at the insertion end, or in the upper connection region (33) of the boundary walls (17, 18) or in the lower region of the end wall (43).

10. The filling rack according to at least one of the preceding claims, **characterized in that** preferably both boundary walls (17, 18) are provided with grip recesses (47) at opposing locations in an upper region facing the upper edges (31) as well as the open front edges (41).

11. The filling rack according to claim 10, **characterized in that** the grip recesses (47) are preferably identically formed elongated rectangular and extending parallel to the open front edge (41) and on both boundary walls (17, 18).

12. The filling rack according to at least one of the preceding claims, **characterized in that** the boundary walls (17, 18) are provided on the inside at least in their approximately parallel upper region (23) with friction decreasing measures such as lubricant coating or the like or are roughened, sand-blasted or the like.

13. The filling rack according to at least one of the preceding claims, **characterized in that** it is formed of impact proof and/or unbreakable plastic, for example PETG, polycarbonate, PP, polystyrene, ABS, acryl.

14. The filling rack according to claim 13, **characterized in that** crystal clear mat, opaque or color tinted plastic or structured plastic is used.

15. The filling rack according to at least one of the preceding claims, **characterized in that** it is injected or formed by a deep-drawn part.

## Revendications

1. Support de remplissage (10) pour bouillottes (11), comprenant un fond (16) et deux parois latérales de limitation (17, 18) qui sont disposées à distance l'une de l'autre et reliées au fond (16) et dont l'écartement, depuis ledit fond (16) vers le logement de maintien (32), diminue dans une zone inférieure (21) et reste constant dans une zone supérieure (23), ainsi qu'un logement de maintien (32) pour la tubulure de remplissage (13) de la bouillotte (11), **caractérisé par le fait que** ledit fond (16) et lesdites parois de limitation (17, 18) sont formés d'un seul tenant et que ledit logement de maintien (32) pour la tubulure de remplissage (13) est formé par des zones d'arête (31) supérieures libres des parois de limitation (17, 18), lesdites zones d'arête (31) étant pourvues d'un évidement (37, 38) à peu près en forme de rigole.

2. Support de remplissage selon la revendication 1, **caractérisé par le fait que** ladite zone inférieure (21) et ladite zone supérieure (23) des parois de limitation (17, 18) présentent à peu près la même longueur.

3. Support de remplissage selon la revendication 1 ou 2, **caractérisé par le fait que**, dans la direction d'insertion (E) de la bouillotte (11) entre les parois de limitation (17, 18), lesdites zones d'arête (31) des parois de limitation (17, 18) se terminent de manière rectiligne vers le haut.

4. Support de remplissage selon la revendication 1 ou 2, **caractérisé par le fait que**, dans la direction d'insertion (E) de la bouillotte (11) entre les parois de limitation (17, 18), lesdites zones d'arête (31) des parois de limitation (17, 18) sont repliées vers l'intérieur l'une en direction de l'autre.

5. Support de remplissage selon l'une au moins des revendications précédentes, **caractérisé par le fait que** les parois de limitation (17, 18) sont reliées entre elles dans une zone (28) située derrière le logement de maintien (32) dans la direction d'insertion (E) de la bouillotte (11), et que, le cas échéant, la zone supérieure de jonction (33) des deux parois de limitation (17, 18), qui est prévue dans ladite zone arrière (28), est contiguë au logement de maintien (32).

6. Support de remplissage selon l'une au moins des revendications précédentes, **caractérisé par le fait que** ledit logement de maintien (32) est disposé au milieu transversal sur les parois de limitation (17, 18).

7. Support de remplissage selon l'une au moins des revendications précédentes, **caractérisé par le fait que** les parois de limitation (17, 18) et le fond (16) sont reliés entre eux, sur une face frontale (42) opposée à la face frontale ouverte d'insertion (41), par l'intermédiaire d'une paroi frontale (43), le fond (16) et les parois de limitation (17, 18) ainsi que la paroi frontale (43) étant formés de préférence d'un seul tenant.

8. Support de remplissage selon l'une au moins des revendications précédentes, **caractérisé par le fait qu'**une ouverture d'introduction (45) pour un moyen de fermeture de la bouillotte (11) est prévue.

9. Support de remplissage selon la revendication 8, **caractérisé par le fait que** ladite ouverture d'introduction (45) est prévue dans la zone inférieure (21) de l'une ou des deux paroi(s) de limitation (17, 18), de préférence à l'extrémité d'insertion, ou dans la zone supérieure de jonction (33) des parois de limitation (17, 18) ou dans la zone inférieure de la paroi frontale (43).

10. Support de remplissage selon l'une au moins des revendications précédentes, **caractérisé par le fait que**, de préférence, les deux parois de limitation (17, 18) sont munies d'évidements de préhension (47) situés à des endroits en regard l'un de l'autre, dans une zone supérieure montrant vers les arêtes supérieures (31) ainsi que vers les arêtes frontales ouvertes (41).

11. Support de remplissage selon la revendication 10, **caractérisé par le fait que** lesdits évidements de préhension (47) sont réalisés de manière à être rectangulaires oblongs et à s'étendre parallèlement à l'arête frontale ouverte (41) et, de préférence, de façon identique sur les deux parois de limitation (17, 18).

12. Support de remplissage selon l'une au moins des revendications précédentes, **caractérisé par le fait que** les faces intérieures desdites parois de limitation (17, 18) sont pourvues, au moins dans leur zone supérieure (23) à peu près parallèle, de mesures diminuant la friction, tel qu'un revêtement en agent antifriction ou similaire, ou sont rendues rugueuses, sablées ou similaire.

13. Support de remplissage selon l'une au moins des revendications précédentes, **caractérisé par le fait qu'**il est réalisé dans une matière plastique résistante au choc et/ou à la rupture, par exemple en PETG, en polycarbonate, en PP, en polystyrène, en ABS, en acrylique.

14. Support de remplissage selon la revendication 13, **caractérisé par le fait que** l'on met en oeuvre une matière plastique claire, mate, opaque ou teintée d'une couleur ou une matière plastique structurée.

15. Support de remplissage selon l'une au moins des revendications précédentes, **caractérisé par le fait qu'**il est moulé par injection ou constitué par une pièce emboutie.
